# EUROPEAN PATENT SPECIFICATION

(11) **EP 1 328 137 B1**
(45) Date of publication and mention of the grant of the patent: **18.04.2007**
(21) Application number: 03250206.4
(22) Date of filing: 14.01.2003
(51) Int. Cl.: H05B 3/34

(54) **Electric heating/warming fabric articles**
Elektrische heizende/wärmende Stoffgegenstände
Articles textiles chauffants/réchauffants électriquement

(30) Priority: 14.01.2002 US 386180
(43) Date of publication of application: 16.07.2003
(60) Divisional application: 07003172.9
(73) Proprietor: MALDEN MILLS INDUSTRIES, INC., Lawrence, Massachusetts 01842 (US)
(72) Inventor: Rock, Moshe, Brookline, Massachusetts 02446 (US); Sharma, Vickram, Stoneham, Massachusetts 02180 (US)
(74) Representative: Murray, Elisabeth Anne

(56) References cited:
- EP-A- 1 021 064
- DE-A- 3 334 744
- GB-A- 581 212
- GB-A- 587 189
- GB-A- 653 641
- US-A- 4 764 665

## Description

### TECHNICAL FIELD

This application claims benefit from U.S. Application No. 60/386,180, filed January 14, 2002, now abandoned.

The invention relates to electrical resistance heating/warming textile articles.

### BACKGROUND

Techniques known for augmenting heating/warming capabilities of clothing fabric include adding electric wires to the fabric, typically by incorporating the wires directly into the fabric or by attaching the wires to the fabric, e.g., by sewing. It is also known, e.g., from Gross et al. U.S. Patent No. 4,021,640, to print an electrical circuit with a resistance heating element on a plastic sheet, such as MYLAR^{®}, and to incorporate strips of the plastic sheet into a fabric article, such as a glove.

EP 1021064 describes an electric heating/warming composite fabric article having a fabric layer and an electric heating/warming element disposed at the inner surface of the fabric layer.

### SUMMARY

According to an aspect of the invention, there is provided a heating/warming fabric article comprising:
a fabric body having first and second broad surfaces; and
an electrically conductive circuit attached to one of the first and second broad surfaces for producing localized heating of the fabric article upon application of electrical current to said circuit; characterised in that:
   the electrically conductive circuit is formed as an electrically conductive textile of conductive fibres die-cut to the form of the circuit.

Preferred features of the method are defined in the corresponding dependent claims. Using a conductive textile to form the circuit pattern provides a robust, flat, and pliable heating/warming element that can be easily manufactured and readily attached to a textile to form a fabric article. The flexible nature of the conductive textile provides good dexterity when the heating/warming element is used in a glove or other article of clothing in which flexibility is useful. The conductive textile can also be readily die-cut in various circuit patterns and geometries, e.g., to provide differential heating to different areas of an article, as will be discussed further below.

The attaching step may include joining the conductive layer and fabric body with adhesive. The term "adhesive," as used herein, refers to any material that will join the layers, including both liquid adhesives and non-liquid, flowable materials such as hot melt webs (commercially available, e.g., from Bostik Co.).

The method may further include forming an article of clothing including the fabric body. The forming step may include shaping the circuit pattern to conform to the shape of the article of clothing. The article of clothing may be selected from the group consisting of gloves, socks, sweaters, jackets, shirts, pants, hats and footwear.

In some implementations, by varying the effective electricity-conducting volume, e.g., the cross-sectional area, of the heating/warming element in selected regions, the level of heat generation can be controlled. (For heating/warming elements of uniform thickness, the effective volume is typically adjusted by variation of the width and/or length.) For example, in a heating/warming element of the invention for use in a shoe, the volume of the heating/warming element in the region of the toes may preferably be less than its volume in the heel region, thus creating greater resistivity in the region of the toes and greater heat generation. Similarly, for use in gloves, the effective volume of the heating/warming element in the region of the fingers will preferably be less (for greater resistivity and heat generation) than in the palm region.

Preferably, the method further includes configuring the circuit pattern to include areas of relatively higher resistivity and areas of relatively lower resistivity to provide predetermined regions of relatively higher and relatively lower localized heating. The predetermined areas of relatively higher and relatively lower resistivity may be provided by varying the cross-sectional area of one or more selected regions of the circuit pattern. Preferably, the predetermined areas of relatively higher and relatively lower resistivity are provided by varying the conductivity of one or more selected regions of the conductive layer. Preferably, the electric heating/warming article is incorporated into an article of clothing, and the method further includes configuring the circuit pattern to place the areas of relatively higher resistivity adjacent a wearer's extremities when the article of clothing is worn, and/or to place the areas of relatively higher resistivity adjacent regions of the wearer's body where blood flow is close to the skin surface when the article of clothing is worn. This allows more heat to be delivered to the extremities, which are prone to vasorestriction in cold weather.

The method may also include interposing a barrier layer between the fabric body and the sheet-form conductive layer, e.g., by attaching an outer surface of the barrier layer to the fabric layer, and attaching an inner surface of the barrier layer to the sheet-form conductive layer. The attaching steps may include joining the layers with adhesive. A barrier is generally used if wind protection is desired.

Preferably, the method further includes connecting the circuit pattern to a power source, to generate heating/warming. Preferably, the method further includes incorporating the electric heating/warming fabric article into a home textile article, e.g., a blanket, throw, mattress cover or sleeping bag.

In another aspect, the invention features a heating/warming fabric article comprising:
a fabric body having first and second broad surfaces; and
an electrically conductive circuit attached to one of the first and second broad surfaces for producing localized heating of the fabric article upon application of electrical current to said circuit; characterised in that:
   the electrically conductive circuit is formed as an electrically conductive textile of conductive fibres die-cut to the form of the circuit.

Some implementations of this aspect of the invention include one or more of the following features. The fabric layer includes a textile material selected from the group consisting of weft knitted materials, warp knitted materials, woven materials, and nonwoven materials. The fabric layer may have a smooth surface, a raised surface, or a brushed surface. The fabric article is an article of clothing. The fabric article is a blanket. The article of clothing includes an article selected from the group consisting of gloves, socks, sweaters, jackets, shirts, pants, hats, footwear, accessories such as ear muffs and neck warmers, and braces and pads such as medical braces, medical bands, knee pads, back pads, and joint pads.

The circuit pattern may include areas of relatively high resistivity and areas of relatively low resistivity to provide predetermined regions of relatively higher localized heating and predetermined regions of relatively lower localized heating. The areas of relatively higher and relatively lower resistivity may include regions of relatively lesser and relatively greater cross-sectional area, respectively. The fabric article may include an article of clothing, and the circuit may be configured to place the areas of relatively higher resistivity adjacent a wearer's extremities when the article of clothing is worn, and/or to place the areas of relatively higher resistivity adjacent regions of the wearer's body where arteries are close to the skin surface when the article of clothing is worn.

The fabric article may further include adhesive interposed between the conductive textile layer and fabric body. The fabric article may further include a barrier layer between the fabric layer and conductive textile layer. The fabric layer, conductive textile layer, and barrier layer (if present) may be joined by adhesive.

Preferably, the fabric article further includes a temperature sensor for measuring the temperature of a portion of the circuit pattern. The temperature sensor is configured to measure the temperature of a first portion of the circuit pattern, and the first portion of the circuit pattern is configured to have the same resistance as a second portion of the circuit pattern, to allow the temperature of the second portion to be estimated by measuring the temperature of the first portion. The fabric article may further include a controller configured to adjust the power supplied to the circuit pattern in response to changes in the measured temperature.

In the above mentioned method a first portion of the conductive layer may be relatively narrower to increase localized heating and a second portion of the conductive layer may be relatively wider to decrease localized heating. The circuit pattern may be attached to an outer surface of a fabric body and the fabric body may be incorporated into an article of clothing. A power source may be connected to the circuit pattern, thereby producing localized heating of the fabric body upon application of electrical current to the circuit pattern. Preferably, the second portion of the conductive layer may be made sufficiently wide that the second portion does not heat up at all, and functions only as a bus.

The details of one or more embodiments of the invention are set forth in the accompanying drawings and the description below. Other features, objects, and advantages of the invention will be apparent from the description and drawings.

### DESCRIPTION OF DRAWINGS

FIGS. 1 and 1A are somewhat diagrammatic exploded side edge views of the components forming the first embodiments of a heating/warming composite fabric article constructed in accordance with the invention;
FIG. 2 is a somewhat diagrammatic side edge view of the heating/warming composite fabric article of FIG. 1; and
FIGS. 3, 4 and 5 are somewhat diagrammatic front plan views of the inner surfaces of heating/warming composite fabric articles of FIGS. 1 and 2, with electric heating/warming elements affixed thereupon, e.g., for a glove (FIG. 3), for an article of footwear (FIG. 4), and for a garment such as a shirt or jacket (FIG. 5); and
FIG. 6 is a somewhat diagrammatic front view of a garment, i.e., a jacket, incorporating the heating/warming composite fabric article of FIG. 5.
FIG. 7 is a somewhat diagrammatic exploded side edge view of the components forming another embodiment of a heating/warming composite fabric article constructed in accordance with the invention; and
FIG. 8 is a somewhat diagrammatic side edge view of the heating/warming composite fabric article of FIG. 7.
FIG. 9 is a somewhat diagrammatic side edge view of another embodiment of a heating/warming composite fabric article constructed in accordance with the invention.
FIGS. 10 and 11 are sequential, somewhat diagrammatic front plan views of the inner surface of a heating/warming composite fabric article during construction in accordance with another embodiment the invention.
FIG. 12 is a somewhat diagrammatic exploded side edge view of the components forming another embodiment of a heating/warming composite fabric article constructed in accordance with the invention, while FIGS. 13 and 14 are somewhat diagrammatic side edge views of alternate embodiments of the heating/warming composite fabric article of FIG. 12.
FIGS. 15 and 16 are somewhat diagrammatic front plan views of an electric heating/warming element for use in a glove.
FIG 17 is a somewhat diagrammatic front plan view of an electric heating/warming element for use in a glove, including a temperature sensing element.
FIG 18 is a somewhat diagrammatic front plan view of an electric heating/warming element that includes a parallel circuit.
FIG. 19 is a somewhat diagrammatic front plan view of an electric heating/warming element for use in an article of footwear.

Like reference symbols in the various drawings indicate like elements.

### DETAILED DESCRIPTION

This application relates to the disclosure of our prior co-pending patent applications U.S. Application No. 09/298,722, filed April 23, 1999, U.S. Application No. 09/389,761, filed September 9,1999, U.S. Application No. 60/175,202, filed January 10, 2000, U.S. Application No. 60/261,544, filed January 12, 2001, and U.S. Application No. 60/386,180, filed January 14, 2002.

According to one preferred embodiment of the invention, the heating/warming element 16 consists of die cut conductive textile, through which an electric current is conducted for producing local heating. The conductive textile may be, for example, a knitted, woven or non-woven material containing conductive fibers or yarns, or a metallized textile . The heating/warming element may be incorporated, e.g., directly or in the form of a textile laminate, into articles of clothing or footwear, and into home furnishings such as blankets and the like. Electric current, e.g. alternating current, via a power cord and plug, or direct current, via a battery, is then applied through the element to cause generation of heat, due to electric resistance.

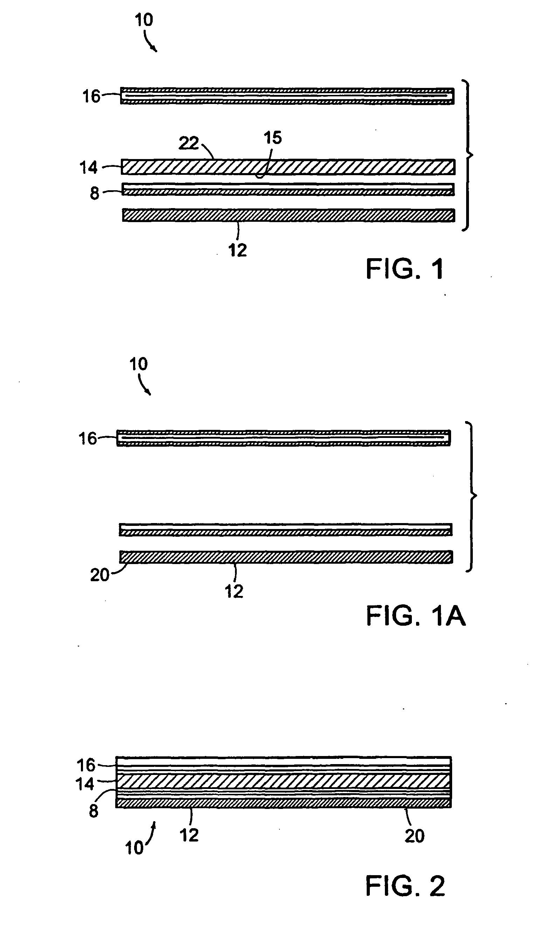

Referring first to FIGS. 1 and 2, in a first embodiment, a windproof, water-resistant, and vapor permeable electric heating/warming composite fabric article 10 constructed in accordance with this invention has three major components. These components include a fabric layer 12, a barrier layer 14 and an electric heating/warming element 16, the fabric layer 12 and barrier layer 14 being joined at opposed fabric inner surface 13 and barrier outer surface 15, respectively, by adhesive 18.

Referring to FIG.1A, in another embodiment the barrier layer 14 may be omitted. In this case, the electric heating/warming composite fabric article includes a fabric layer 12 and an electric heating/warming element 16. The fabric layer 12 inner surface 13 is joined to the inner surface 17 of the electric heating/warming element 16 by adhesive 18.

In both of the embodiments shown in FIGS. 1 and 1A, the fabric article 10 may further include a second fabric layer (not shown), with the heating/warming element and the barrier layer (if included) being interposed between the two fabric layers.

Referring to FIG. 1, 1A, and 2, in preferred embodiments, the fabric layer 12 is made in any well known manner, e.g. the fabric layer 12 may be a knitted material, e.g., a plaited circular knitted or reverse plaited circular knitted material, or other circular knitted material (such as double knitted, single jersey knitted, two-end fleece knitted, three-end fleece knitted, terry knitted or double loop knitted material), or warp knitted or weft knitted material, or a woven or non-woven material. In applications where the fabric layer 12 of the fabric article 10 will be directed outwardly, away from the wearer's skin, the material of the fabric layer is preferably hydrophobic, in order to resist penetration of liquids. In other applications, where the fabric layer 12 of the fabric article 10 will be directed inwardly, toward the wearer's skin, the material of the fabric layer is preferably naturally hydrophilic, chemically rendered hydrophilic, or hydrophobic, in order to enhance removal and transport of perspiration away from the skin. The inner surface 13 of fabric layer 12, to which the adhesive 18 is adhered, is preferably flat. The exposed, outer surface 20 of fabric layer 12 may be flat or raised, e.g. by brushing, sanding or napping, and/or may be otherwise provided with decorative and functional features and finishes, e.g. as well known in the art.

Preferably, the barrier layer 14 is formed of a vapor permeable membrane which is nonporous hydrophilic or micro-porous hydrophobic or a combination of both, e.g. in layers, as appropriate to the nature of the intended use, or as otherwise desired. In certain embodiments, it may also be preferred that the material of the barrier layer 14 be soft and stretchable. The barrier layer is constructed and/or formulated to resist air and water droplets from passing through the composite fabric article 10 while being permeable to water vapor. In applications where it is desired that the fabric article 10 is stretchable, the fabric layer 12 may typically be a knitted material, and a preferred material for barrier layer 14 is poly urethane, e.g. as available from UCB Chemical Corp. of Drogenbos, Belgium, either micro-porous hydrophobic (preferred for use where the barrier layer 14 is directed outward) or nonporous hydrophilic (preferred for use where the barrier layer 14 is directed inward). Alternatively, in situations where relatively less stretch is required, e.g. in footwear, the fabric layer 12 may be a warp knitted material, and a preferred material for barrier layer 14 is poly tetrafluoroethylene (PTFE), e.g., as available from Tetratec, of Feasterville, Pennsylvania.

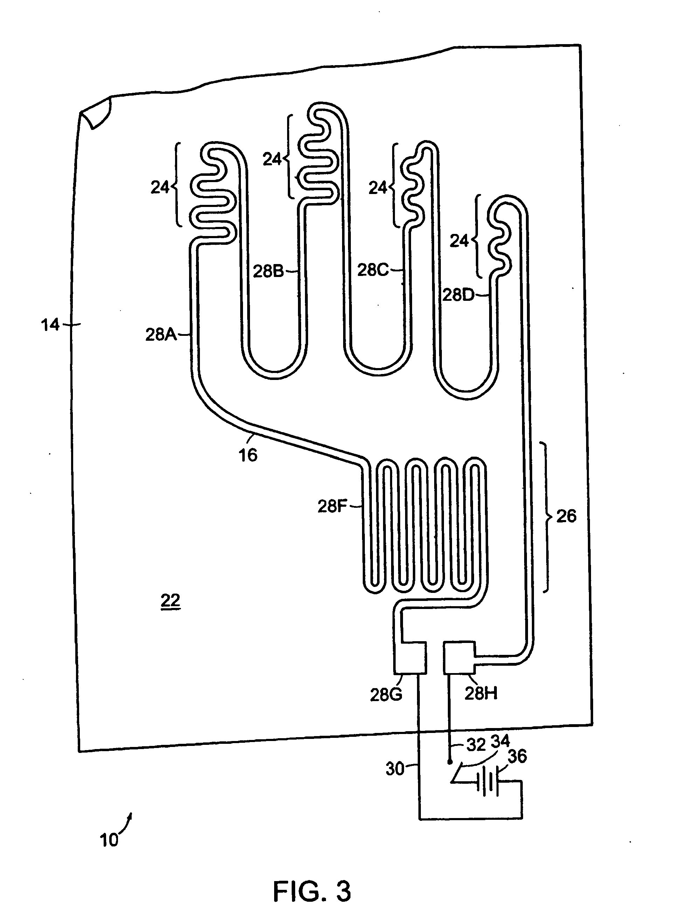

The barrier layer 14 is joined to the inner surface 13 of fabric layer 12 by adhesive 18, typically applied in spots, lines or other discrete regions, or by attachment, lamination or other suitable manner of combining. A similar composite fabric (but having an additional internal fabric layer) is described in commonly assigned Lumb et al. U.S. Patent No. 5,364,678, the entire disclosure of which is incorporated herein by reference. Referring also to FIG. 3, electric heating/warming element 16 is disposed upon the outer surface 22 of barrier layer 14.

In one embodiment, the electric heating/warming element 16 is formed of metallized textile. Suitable metallized textiles are available, e.g., from Schlegel Systems Inc. of Rochester, New York. The textile may be metallized by any suitable technique, e.g., by metal coating, plating, or deposition, using chemical, electrical or mechanical techniques. The metal coating or deposit is made of a conductive material that provides a very low resistance, typically less than about 500 ohms per square. Examples of suitable conductive materials include silver, copper, nickel, nickel-chrome, and combinations of these metals. The metallized textile can be produced in sheets and then diecut into the desired pattern. The element (or its parts) is then attached or inserted, e.g., alone or laminated to or between one or two layers of suitable non-conductive material, to, or into, the fabric layer 12, to form a textile product. For a textile article in the form of a blanket, formation of the electric heating/warming element as a die cut stamping allow the buses to be formed integrally with the heating elements. The heating elements may be spaced asymmetrically so that selected regions get preferentially warmer than other regions, or, as described in more detail below, by providing selected heating elements that are relatively more narrow than other heating elements, greater resistivity, with resultant generation of more heat, can be provided to selected regions.

Alternatively, the heating/warming element may be formed of a conductive textile, e.g., a textile that includes conductive fibers and/or yarns. Suitable conductive fibers and yarns include, for example, carbon and polyaniline.

The predetermined pattern of the heating/warming element 16 may be custom designed for the particular purpose for which the composite fabric article 10 of the invention is to be used. For example, the pattern of the heating/warming element 16 of the composite fabric article 10 of FIG. 3 is designed for use in making a glove. For this purpose, the electric heating/warming element 16 forms a pattern having four elongated branches 28A, 28B, 28C, 28D (corresponding to fingers of a glove) and one or more sections 28F (corresponding to the palm or back of the body of a glove).

The heating/warming element 16 is formed as a continuous circuit, terminating at each end in a contact pad 28G, 28H, respectively. The contact pads preferably are disposed adjacent to each other in a region convenient for connection to a source of power, e.g. for a glove, as shown, in a region to form the wrist of the glove. Still referring to FIG. 3, the heating/warming element 16 is connected, by wire conductors 30, 32 extending from contact pads 28G, 28H, respectively, in a circuit including a switch 34 and a power supply, e.g., a battery pack 36. When switch 34 is closed, the heating/warming element 16 is activated to generate heat/warmth.

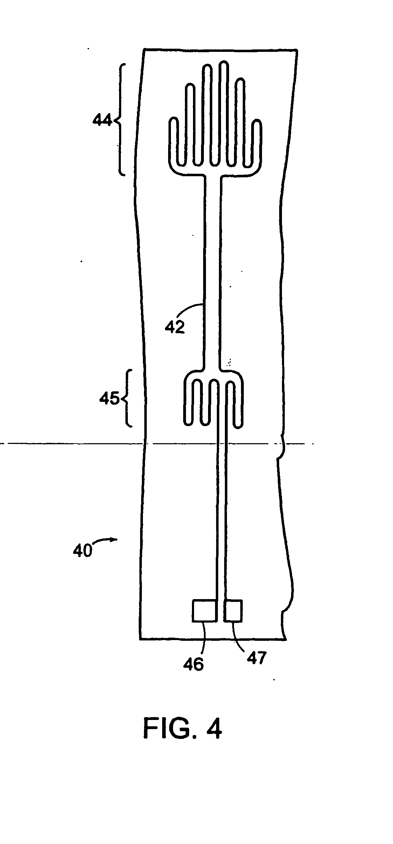

The pattern features of the heating/warming element 16 shown in FIG. 3 are sized and shaped to conform to the regions of the resulting fabric article, i.e., the glove, so that the composite fabric can readily be cut to form one side of a glove. Patterns for use in other types and sizes of garments and fabric articles, e.g. such as socks, sweaters, jackets, shirts, pants, hats, gloves, footwear (e.g. shoes and boots) and so on, can be generated in a similar manner, e.g., as will be discussed below with reference to FIGS. 4-6.

Referring to FIG. 4, a composite fabric article 40 of the invention has a heating/warming element 42 sized and shaped to conform to the regions of the selected resulting fabric article, i.e., in this embodiment, a boot, to be heated/warmed so that the composite fabric can readily be cut to be formed and/or incorporated into a boot liner. In particular, the heating/warming element 42 has heating/warming regions 44, 45, with sections of relatively reduced cross-sectional area for increased resistivity and heat generation, corresponding to the toe/ball and heel surfaces, respectively, of a wearer's foot. -The heating/warming element 42, which forms a circuit, terminates at each end in a contact pad 46, 47, respectively. The contacts pads are disposed adjacent to each other in a region convenient for connection to a source of power, e.g., as shown, in a region to extend into or above the ankle collar of the boot.

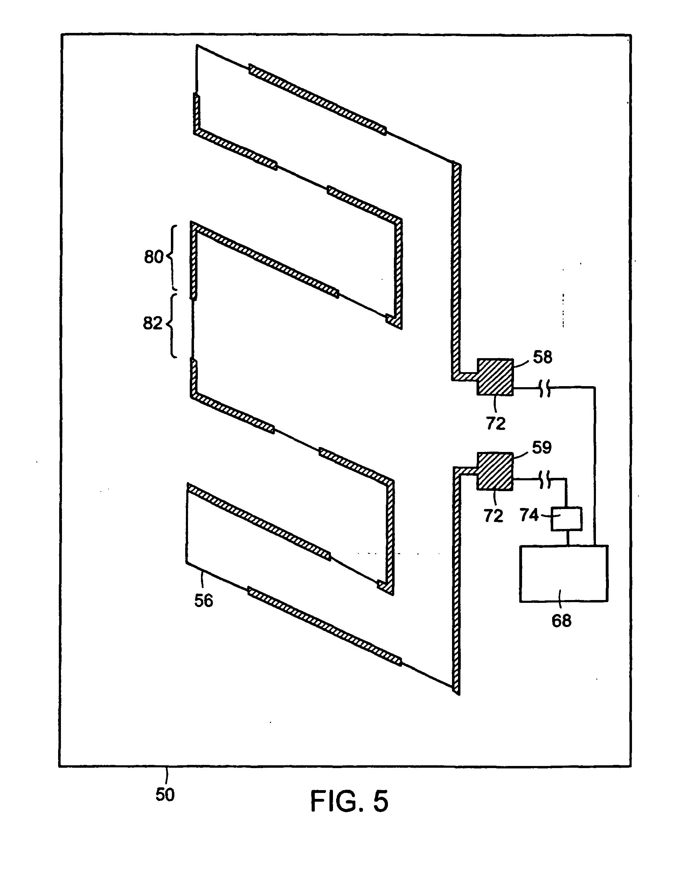

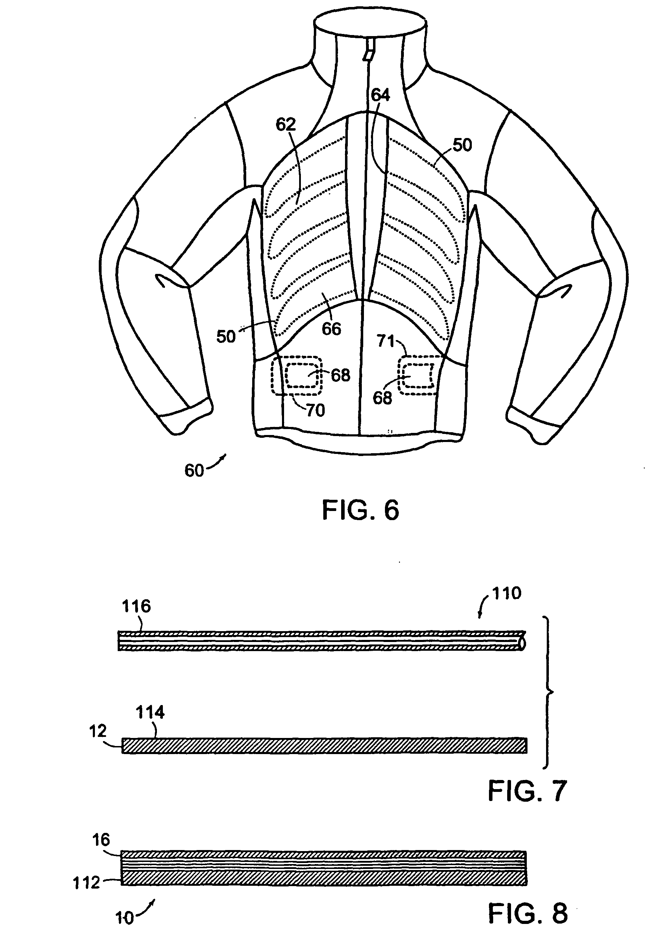

Referring to FIG. 5, a composite fabric article 50 of the invention has a heating/warming element 56 sized and shaped to conform to the regions of the selected resulting fabric article, i.e., in this embodiment, the opposite chest surfaces of a garment such as a shirt or a jacket 60 (FIG. 6), to be heated/warmed. The heating/warming element 56 terminates at each end in a contact pad 58, 59, respectively, the pads being disposed adjacent to each other in a region convenient for connection to a source of power, as discussed below.

Referring also to FIG. 6, a pair of fabric articles 50 is shown incorporated into jacket 60. A battery pack 68 for powering each of the heating/warming composite fabric articles 50 is contained in the associated zippered pockets 70, 71. The battery pack 68, e.g. as available from Polaroid Corporation, of Cambridge, Massachusetts, is preferably removably connected to the contact pads 58, 59 of heating/warming element 56 by releasable fastening elements 72, e.g. clips, snaps or other secure but releasable fastening elements. (The fastening elements may provide the electrical connection of the battery pack to the circuit, or, alternatively, may maintain the battery pack in position for contact of the battery pack with separate connectors.) This arrangement permits the battery pack 68 to be removed, e.g., whenever the fabric article 50 is to be washed, or for replacement. The heating/warming circuit 56 may also include an oscillator chip 74 or other timing or cycling device for cycling application of electrical power from the battery pack 68 to the heating/warming element 56, e.g., to extend battery pack life. For example, a timing cycle of three minutes "on" followed by one minute "off" is considered suitable for an electric heating/warming composite fabric article 50 incorporated as a chest panel of the heating/warm jacket 60 suited for outdoors use.

In one preferred embodiment, a composite fabric article 10 of the invention is formed by first combining the fabric layer 12 and barrier layer 14 with adhesive 18 disposed therebetween. An electric heating/warming element 16 is then affixed upon the surface 22 of the barrier layer 14. The resulting composite fabric article 10 is cut to shape, and otherwise processed using standard clothing procedures, for incorporation, e.g., into an article of clothing or the like. Alternatively, the heating/warming element 16 may be affixed upon the surface 22 of the barrier layer 14, before the barrier layer 14 and the fabric layer 12 are secured together.

Referring next to FIGS. 7 and 8, in another embodiment of the invention, an electric heating/warming composite fabric article 110 consists of a fabric layer 112 having an inner surface 114 upon which an electric heating/warming element 116 is disposed.

In embodiments of the invention where the heating/warming element 116 is affixed directly to the fabric layer 112, the composite fabric article 110 may be employed without a barrier layer. Alternatively, a pair of fabric articles 110 may be incorporated into a garment, e.g. a jacket 60, as shown in FIG. 6, where the outer coverings 62, 64 of the opposite chest surfaces of the jacket may be a shell material selected to provide a barrier laver overlaying the heating/warming composite fabric articles 110 incorporated into the jacket.

The relative amounts of heat/warmth generated by a region of an electrical heating/warming element in a composite heating/warming fabric article of the invention can be controlled, e.g., by varying the width and/or by varying the length and/or the thickness of a circuit element or segment, and/or by varying the conductivity/resistivity of the material forming a segment of the circuit element.

For example, referring to FIG. 5, a heating/warming element 56 formed of material of uniform conductivity and constant thickness has regions 80 and 82 of contrasting width, and, therefore, contrasting cross sectional area. As a result, in region 80 of relatively greater width, there is more conductivity, i.e. less resistance to current flow, and thus less generation of heat/warmth. Similarly, in region 82 of relatively lesser width, there is less conductivity, i.e. more resistance to current flow, and thus relatively greater generation of heat/warmth. As a result, a composite heating/warming fabric article 50 of the invention can be designed with a circuit element 56 that delivers relatively greater amounts of heat/warmth to selected regions of the wearer's body.

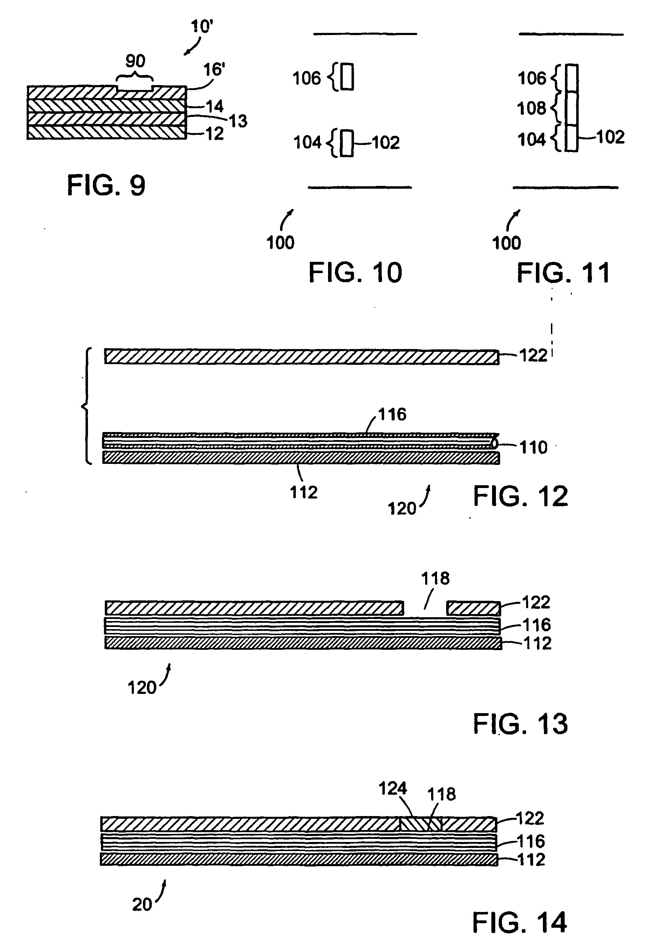

Alternatively, this effect may be obtained by applying a thinner layer of material, i.e., a region of relatively lesser cross sectional area. For example, referring to FIG. 9, a composite heating/warming fabric article 10' of the invention has a heating/warming element 16' having a region 90 of relatively lesser thickness (compared to adjacent regions).

Alternatively, or in addition, a heating/warming element of constant dimension but with regions generating relatively different levels of heat/warmth may be formed by sequentially applying circuit regions using materials of inherently different conductivity. For example, referring first to FIG. 10, showing a composite heating/warming fabric article 100 of the invention, a heating/warming element 102 is formed by affixing regions 104, 106 of a material of relatively greater conductivity, and thereafter, referring to FIG. 11, affixing region 108 of a material of relatively lower conductivity, region 108 interconnecting regions 104, 106.

These and other methods for adjusting the conductivity of electrical circuit regions may be employed alone, or in any desired combination.

The conductivity of various regions of the electrical circuit may be adjusted to suit the requirements of a particular application and thereby enhance wearer comfort. For example, in the case of gloves or footwear, heating the extremities (fingers and toes) is important to providing comfort, and generally the fingers and toes, especially at their tips, require more heating than the rest of the hands and feet. Thus, it is may be desirable to generate more heat in these specific areas, which may be accomplished in any of the manners discussed above.

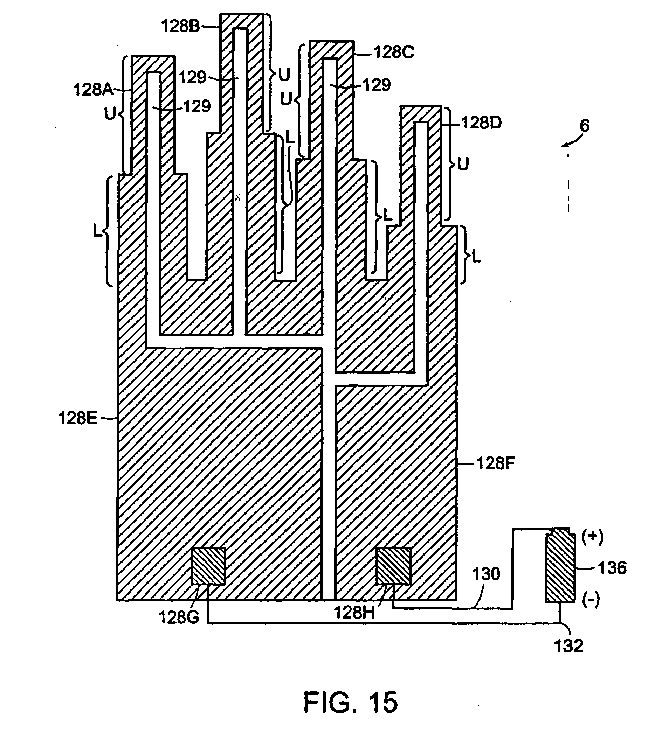

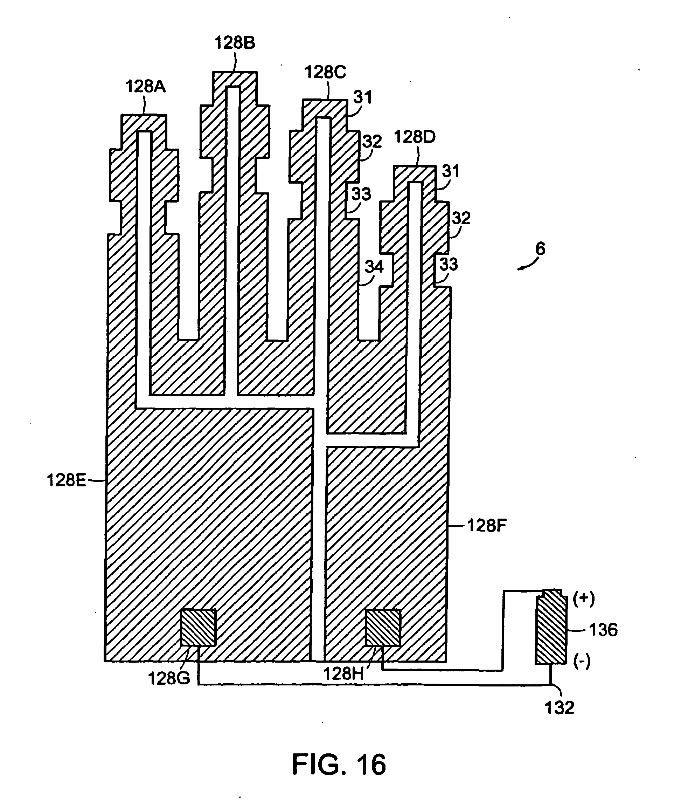

Preferred heating elements for use in gloves are shown in FIGS 15 and 16. In both of these embodiments, the electric heating/warming element 116 forms a pattern having four elongated branches 128A, 128B, 128C, 128D (corresponding to fingers of a glove) and sections 128E and 128F (corresponding to the palm or back of the body of a glove). A region 129 is cut out, or is not metallized, to reduce the effective area of the conductive material. The presence of region 129 increases the resistivity of the branches 128A-128D, while not significantly affecting the conductivity of the palm sections 128E and 128F. As a result, more heat will be generated in the branches 128A-128D than in the palm sections.
Additionally, within the branches 128A-128D there are regions of different width. For example, in the embodiment shown in FIG. 15, the branches 128A-128D include upper regions U, generally corresponding to the portion of the wearer's fingers from the first knuckle to the tip, and lower portions L, generally corresponding to the portion of the wearer's fingers from the first knuckle to the intersection of the finger with the palm. The upper regions U are narrower than the lower regions L, and thus have a greater resistivity and as a result generate more heat at the wearer's fingertips.

When the pattern shown in FIG 15 is powered by 3.0 volts direct current source with an element having a resistance of 4.8 Ohms, the temperature generated in upper portions U is about 101 degrees Fahrenheit while the temperature generated in lower portions L is about 80 degrees Fahrenheit. This provides greater heat generation in the fingers, and particularly at the tips of the fingers, providing more comfort for the user while conserving battery power.

Similarly, in the embodiment shown in FIG. 16, the width of the branches 128A-128D is further varied, to provide narrow areas 31 and 33, generally corresponding, respectively, to the tips and first knuckles of a wearer, and wide areas 32 and 34, generally corresponding to the areas between the knuckles of the wearer. In this example when the element is powered by a 3.0 volts direct current source with the element having a resistance of 4.8 Ohms, the temperature generated at narrow areas 31 and 33 is about 101 degrees Fahrenheit, while the temperature generated at wide areas 32 and 34 is about 80 degrees Fahrenheit. The section next to the terminals and in the palm area will have very low resistance and thus will generate very little, if any, heat. Thus, the narrow areas 31 and 33 provide high heat generation at the fingertips and close to the arteries (at the first knuckle). Providing heat generation at regions close to arteries helps to warm the blood and improve circulation. As a result, the user's fingers are kept warm without overheating the rest of the user's hand, while also conserving battery power.

In the embodiments shown in Figs. 15 and 16, power is delivered to the circuit in the same manner as discussed above with reference to Fig. 3. That is, the heating/warming element 16 is formed as a continuous circuit, terminating at each end in a contact pad 128G, 128H, respectively, for connection to a source of power, e.g., a battery pack 136, by wire connectors 130, 132.

In yet another embodiment of the invention, the electric heating/warming composite fabric article 110 described above with reference to FIGS. 5 and 6 may be further processed. For example, referring now to FIGS. 12, 13 and 14, in an electric heating/warming composite fabric article 120, a barrier layer 122, e.g. as described above, is attached adjacent to the side of the inner surface 114 of the fabric layer, overlying at least a portion of the heating/warming element 116, using adhesive, also as described above. Preferably, contact pads 118 (only one is shown) of heating/warming element 116 are left exposed for connection to a source of power (FIG. 13), or electrical connectors 124 (only one is shown) are provided for connecting the contact pads and power source through the barrier layer 122 (FIG. 14).

In all cases described above, the heating/warming element is supported by a fabric layer, whether or not a barrier layer is provided. The fabric layer may be naturally hydrophilic, chemically rendered hydrophilic, or hydrophobic. In some preferred embodiments, a barrier layer is provided at least adjacent to the inner surface of the fabric layer, i.e., attached to the fabric layer (with or without intervening materials) or spaced from attachment to or upon the fabric layer, but positioned at the inner surface side of the fabric.

A barrier layer associated with or attached, e.g. by lamination or other techniques, upon the surface of the fabric layer 12 upon which the heating/warming element 16 is affixed (e.g. barrier layers 62, 64; FIG. 6 and barrier layer 122; FIGS. 12-14, respectively) serves also to protect the circuit against the effects of abrasion that might otherwise deteriorate the quality or continuity of the electrical heating circuit. The barrier layer would also serve to resist short-circuiting in the event that condensate forms on the fabric layer inner surface. The barrier layer may be formed of any suitable, protective material. It will preferably be micro porous hydrophobic or nonporous hydrophilic if it is a complete layer. Where a complete layer is not desired or employed, the barrier layer may be applied exclusively to the printed circuit itself, in which case, it will preferably be nonporous hydrophobic.

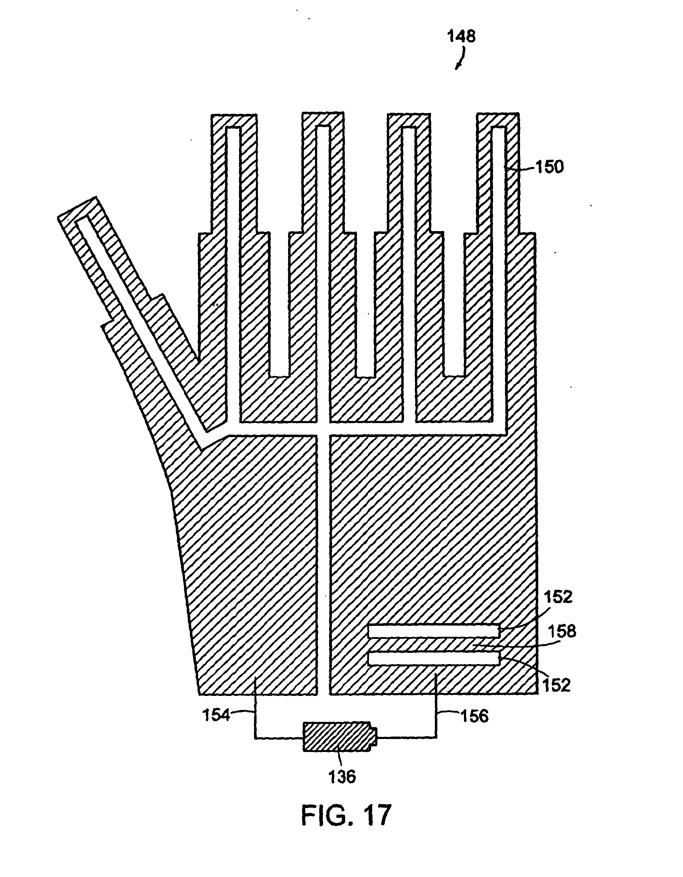

If desired, the temperature of a portion of the heating/warming element can be measured during use. For instance, a sensor can be included to determine the temperature at the fingertip of the glove. The sensor can be placed at the fingertip, with a wire running down the finger. However, this may interfere with dexterity, and thus it may be desirable to simulate the fingertip temperature at another area of the glove and measure the temperature at that area. For example, in the heating/warming element 148 shown in Fig. 17, the temperature at fingertip 150 can be simulated by providing two cut-out areas 152 in the palm region, near the wire conductors 154, 156, that define a rectangular area 158 that is calculated to have the same resistance as the portion of the circuit in the fingertip 150. Thus, the temperature at the fingertip can be estimated remotely by measuring the temperature of the area 158. This temperature data can be used, in conjunction with a controller (not shown), e.g., a voltage regulator, to automatically shut off the battery or deliver less power to the circuit when a maximum temperature is detected, and turn on the battery or increase power delivery when a minimum temperature is detected. Alternatively, or in addition, the temperature can be displayed on a read-out (not shown) mounted on the glove, and a manual control can be provided to allow the wearer to turn the battery on and off or adjust the temperature.

A number of embodiments of the invention have been described. Nevertheless, it will be understood that various modifications may be made without departing from the spirit and scope of the invention.

For example, additional fabric layers may be added to enhance various aesthetic and functional characteristics of the electric heating/warming composite fabric article.

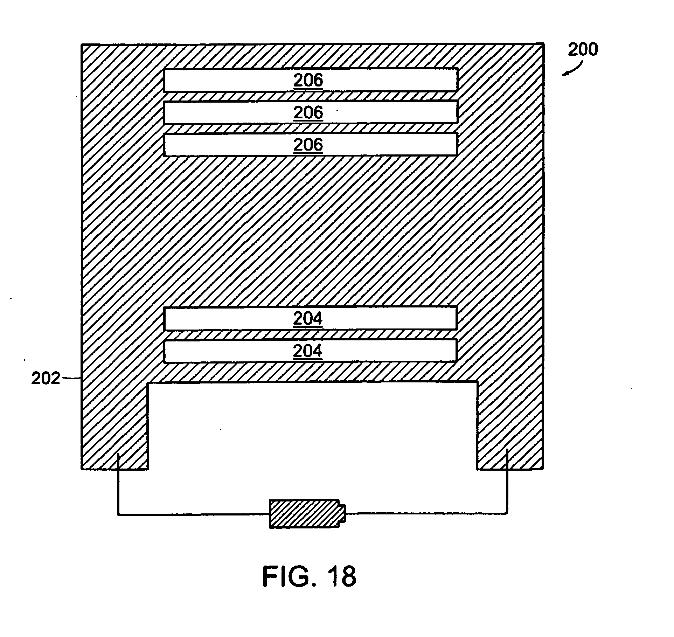

Moreover, while the circuits in the embodiments discussed above have been series circuits, the circuit used in the heating/warming element may be a parallel circuit, e.g., as shown in Fig. 18. In the heating/warming element 200, shown in Fig. 18, the relatively wide areas 202 act as buses, while the cut-out areas 204, 206 provide areas of higher resistivity, as discussed above. The circuit shown in Fig. 18 also illustrates that the circuit need not be symmetrical, e.g., in the circuit shown in Fig. 18 there are three cut-out areas 206 in the upper region of the circuit, but only two cut-out areas 204 in the lower region of the circuit.

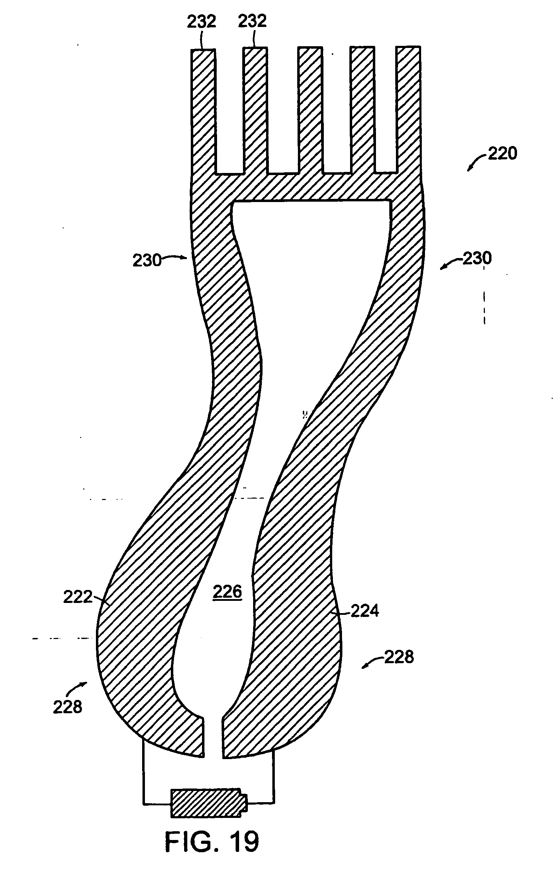

Further, while circuits for gloves have been described above, by way of example, the heating/warming element may be used in many different applications. For example, a heating/warming element 220, for use in a sock, shoe, or other article of footwear, is shown in Fig. 19. In the heating/warming element 220, the circuit includes a left hand portion 222 and a right hand portion 224, separated by a cut-out area 226. Cut-out area 226 is shaped to provide relatively wide bus areas 228 in the heel region, and relatively narrower, higher resistivity areas 230 in the forefoot region. The toe portions 232 are narrowest of all, and thus have the highest resistivity, so that the highest temperature will be generated adjacent the wearer's toes.

Accordingly, other embodiments are within the scope of the invention.

## Claims

1. A method of forming an electric heating/warming fabric article (10), the method comprising:
attaching an electrically conductive circuit (16) to one of a first and a second broad surface of a fabric body (12) for producing localized heating of the fabric body (12) upon application of electrical current to said circuit;
**characterised in that**:
the electrically conductive circuit (16) is formed by die-cutting an electrically conductive textile of conductive fibres.

2. The method of claim 1 wherein said attaching step comprises joining the circuit (16) and fabric body (12) with adhesive (18).

3. The method of any one of the preceding claims further comprising forming an article of clothing (60) including said fabric body (12).

4. The method of claim 3 wherein the forming step comprises shaping the circuit to conform to the shape of the article of clothing (60).

5. The method of claim 3 or 4 wherein the article of clothing (60) comprises an article selected from the group consisting of gloves, socks, sweaters, jackets, shirts, pants, hats, footwear, ear muffs, neck warmers, medical braces, medical bands, knee pads, back pads, and joint pads.

6. The method of any one of the preceding claims further comprising configuring the circuit (16) to comprise areas (82) of relatively higher resistivity and areas (80) of relatively lower resistivity to provide predetermined regions of relatively higher and relatively lower localized heating.

7. The method of claim 6, wherein the predetermined areas (80, 82) of relatively higher and relatively lower resistivity are provided by varying the cross-sectional area of one or more selected regions of the circuit (56).

8. The method of claim 6 or 7, wherein the predetermined areas (80, 82) of relatively higher and relatively lower resistivity are provided by varying the conductivity of one or more selected regions of the circuit (16).

9. The method of claim 6, 7 or 8 wherein the electric heating/warming article (10) is incorporated into an article of clothing (60), and the method further comprises configuring the circuit to place said areas of relatively higher resistivity adjacent a wearer's extremities when the article of clothing is worn.

10. The method of any one of Claims 9 to 12 wherein the electric heating/warming article is incorporated into an article of clothing (60), and the method further comprises shaping the circuit to place said areas of relatively higher resistivity adjacent regions of the wearer's body where blood flow is close to the skin surface when the article of clothing is worn.

11. The method of any one of the preceding claims wherein the fabric body (12) comprises a textile material selected from the group consisting of weft knitted materials, warp knitted materials, woven materials, and non-woven materials; preferably the fabric body (12) has a smooth surface, a raised surface, or a brushed surface.

12. The method of any preceding claim further comprising interposing a barrier layer (14) between the fabric body (12) and the circuit (16).

13. The method of Claim 12 further comprising attaching an outer surface of the barrier layer (14) to the fabric body (12), and attaching an inner surface of the barrier layer (14) to the circuit (16).

14. The method of Claim 13 wherein said attaching steps comprise joining the layers with adhesive (18).

15. The method of any preceding claim further comprising connecting the circuit to a power source (36), to generate heating/warming.

16. The method of any preceding claim further comprising incorporating the electric heating/warming fabric article (10) into a home furnishing textile article.

17. The method of Claim 19 wherein the home textile article comprises a blanket, throw, sleeping bag or mattress cover.

18. The method of any one of the preceding claims wherein said circuit comprises a series circuit.

19. The method of any one of the preceding claims wherein said circuit comprises a parallel circuit.

20. The method of any preceding claim wherein said circuit is attached to an outer surface of a fabric body (12).

21. A heating/warming fabric article (10) comprising:
a fabric body (12) having first and second broad surfaces; and
an electrically conductive circuit (16) attached to one of the first and second broad surfaces for producing localized heating of the fabric article upon application of electrical current to said circuit; **characterised in that**:
the electrically conductive circuit (16) is formed as an electrically conductive textile of conductive fibres die-cut to the form of the circuit.

22. The fabric article of Claim 21 wherein the fabric body (12) comprises a textile material selected from the group consisting of weft knitted materials, warp knitted materials, woven materials, and non-woven materials; preferably the fabric layer has a smooth surface, a raised surface, or a brushed surface.

23. The fabric article of Claim 21 or 22 wherein the fabric article (10) comprises an article of clothing.

24. The fabric article of any one of Claims 21, 22 or 23 wherein the fabric article (10) comprises a blanket, throw, sleeping bag or mattress cover.

25. The fabric article of any one of Claims 21 to 24 further comprising adhesive (18) interposed between the circuit and fabric body.

26. The fabric article of any one of Claims 23 to 25 wherein the article of clothing (60) comprises an article selected from the group consisting of gloves, socks, sweaters, jackets, shirts, pants, hats, footwear, ear muffs, neck warmers, medical braces, medical bands, knee pads, back pads, and joint pads.

27. The fabric article of any one of Claims 23 to 26 wherein the circuit comprises areas (82) of relatively higher resistivity and areas (80) of relatively lower resistivity to provide predetermined regions of relatively higher localized heating and predetermined regions of relatively lower localized heating.

28. The fabric article of Claim 27, wherein the areas (80,82) of relatively higher and relatively lower resistivity comprise regions of relatively lesser and relatively greater cross-sectional area, respectively.

29. The fabric article of any one of Claims 23 to 28 wherein the fabric article (10) comprises an article of clothing (60), and the circuit is configured to place said areas of relatively higher resistivity adjacent a wearer's extremities when the article of clothing is worn.

30. The fabric article of any one of Claims 23 to 29 wherein the fabric article (10) comprises an article of clothing (60), and the circuit is configured to place said areas of relatively higher resistivity adjacent regions of the wearer's body where arteries are close to the skin surface when the article of clothing is worn.

31. The fabric article of any one of Claims 23 to 30, further comprising a barrier layer (14) between the fabric body (12) and the circuit (16).

32. The fabric article of Claim 31 wherein the barrier layer (14), fabric body (12), and circuit (16) are joined by adhesive (18).

33. The fabric article of any one of Claims 21 to 32 wherein said circuit comprises a series circuit.

34. The fabric article of any one of Claims 21 to 33 wherein said circuit comprises a parallel circuit.

35. The fabric article of any one of Claims 21 to 34 wherein said circuit is asymmetrical.

36. The fabric article of any one of Claims 21 to 35 further comprising a temperature sensor for measuring the temperature of a portion of the circuit.

37. The fabric article of Claim 36 wherein said temperature sensor is configured to measure the temperature of a first portion of the circuit, and the first portion of the circuit is configured to have the same resistance as a second portion of the circuit, to allow the temperature of the second portion to be estimated by measuring the temperature of the first portion.

38. The fabric article of Claim 36 or 37 further comprising a controller configured to adjust the power supplied to the circuit in response to changes in the measured temperature.

39. The heating/warming fabric article of claim 21 wherein the conductive textile has a shape corresponding to a selected surface region of a wearer's body and with one or more circuit regions of relatively higher resistivity among one or more circuit regions of relatively lower resistivity, the one or more circuit regions of relatively higher resistivity positioned for correlation with one or more selected heating regions of the wearer's body, the conductive textile, upon application of electrical current to the circuit, producing localized heating in the one or more circuit regions of relatively higher resistivity of the circuit attached upon the fabric body for preferential heating of the one or more selected heating regions of the wearer's body.

40. The heating/warming fabric article of claim 21 wherein the conductive fibers form the electrically conductive circuit.

41. The heating/warming fabric article of claim 21 wherein the conductive fibers comprise a material selected from the group consisting of carbon and polyaniline.

## Patentansprüche

1. Verfahren zur Bilden eines elektrisch heizenden/wärmenden Gewebeartikels (10), wobei das Verfahren aufweist:
Anbringen einer elektrisch leitfähigen Schaltung (16) an einer von einer ersten und einer zweiten breiten Oberfläche eines Gewebekörpers (12), um eine lokale Erwärmung des Gewebekörpers (12) bei Anlegen von elektrischem Strom an die Schaltung zu erzeugen;
**dadurch gekennzeichnet, dass**
die elektrisch leitfähige Schaltung (16) durch Stanzen einer elektrisch leitfähigen Textilie aus leitfähigen Fasern gebildet wird.

2. Verfahren nach Anspruch 1, wobei der Anbringungsschritt ein Verbinden der Schaltung (16) mit dem Gewebekörper (12) durch Haftmittel (18) aufweist.

3. Verfahren nach einem der vorausgehenden Ansprüche, ferner ein Bilden eines Kleidungsartikels (60) aufweisend, der den Gewebekörper (12) einschließt.

4. Verfahren nach Anspruch 3, wobei der Schritt des Bildens ein Formen der Schaltung aufweist, um der Form des Kleidungsartikels (60) zu entsprechen.

5. Verfahren nach Anspruch 3 oder 4, wobei der Kleidungsartikel (60) einen Artikel aufweist, welcher ausgewählt ist aus der Gruppe bestehend aus Handschuhen, Socken, Pullovern, Jacken, Hemden, Hosen, Hüten, Schuhwerk, Ohrenschützern, Nackenwärmern, medizinischen Klammern, medizinischen Bändern, Kniepolstern, Rückenpolstern und Gelenkpolstern.

6. Verfahren nach einem der vorausgehenden Ansprüche, ferner ein Konfigurieren der Schaltung (16) aufweisend, um Flächen (82) mit relativ hohem Widerstand und Flächen (80) mit relativ geringem Widerstand zu umfassen, um vorgegebene Bereiche mit relativ hoher und relativ geringer lokaler Wärmeerzeugung bereitzustellen.

7. Verfahren nach Anspruch 6, wobei die vorgegebenen Flächen (80, 82) mit relativ hohem und relativ geringem Widerstand durch Variieren der Querschnittfläche von einem oder mehreren ausgewählten Bereichen der Schaltung (56) bereitgestellt werden.

8. Verfahren nach Anspruch 6 oder 7, wobei die vorgegebenen Flächen (80, 82) mit relativ hohem und relativ geringem Widerstand durch Variieren der Leitfähigkeit von einem oder mehreren ausgewählten Bereichen der Schaltung (16) bereitgestellt werden.

9. Verfahren nach Anspruch 6, 7 oder 8, wobei der elektrisch heizende/wärmende Artikel (10) in einem Kleidungsartikel (60) eingebunden ist und das Verfahren ferner ein Konfigurieren der Schaltung aufweist, um die Flächen mit relativ hohem Widerstand an Gliedmaßen eines Trägers anliegend anzuordnen, wenn der Kleidungsartikel getragen wird.

10. Verfahren nach einem der Ansprüche 9 bis 12, wobei der elektrisch heizende/wärmende Artikel in einen Kleidungsartikel (60) eingebunden ist und das Verfahren ferner ein Formen der Schaltung aufweist, um die Flächen mit relativ hohem Widerstand an Bereiche des Körpers eines Trägers anliegend anzuordnen, wo sich Blutzirkulation nahe der Hautoberfläche befindet, wenn der Kleidungsartikel getragen wird.

11. Verfahren nach einem der vorausgehenden Ansprüche, wobei der Gewebekörper (12) ein Textilmaterial umfasst, welches ausgewählt ist aus der Gruppe bestehend aus Kulierwirkmaterialien, Kettwirkmaterialien, gewebten Materialien und nicht gewebten Materialien; vorzugsweise weist der Gewebekörper (12) eine glatte Oberfläche, eine raue Oberfläche oder eine gebürstete Oberfläche auf.

12. Verfahren nach einem der vorausgehenden Ansprüche, ferner aufweisend ein Einbringen einer Sperrschicht (14) zwischen dem Gewebekörper (12) und der Schaltung (16).

13. Verfahren nach Anspruch 12, ferner aufweisend ein Anbringen einer äußeren Oberfläche der Sperrschicht (14) an den Gewebekörper (12), und ein Anbringen einer inneren Oberfläche der Sperrschicht (14) an die Schaltung (16).

14. Verfahren nach Anspruch 13, wobei die Anbringungsschritte ein Verbinden der Schichten mit Haftmittel (18) aufweisen.

15. Verfahren nach einem der vorausgehenden Ansprüche, ferner aufweisend ein Verbinden der Schaltung mit einer Stromquelle (36), um ein Heizen/Erwärmen zu erzeugen.

16. Verfahren nach einem der vorausgehenden Ansprüche, ferner aufweisend ein Einbinden des elektrisch heizenden/wärmenden Gewebeartikels (10) in einen Heimeinrichtungs-Textilartikel.

17. Verfahren nach Anspruch 19, wobei der Heimtextilartikel eine Decke, einen Überwurf, einen Schlafsack oder einen Matratzenschoner umfasst.

18. Verfahren nach einem der vorausgehenden Ansprüche, wobei die Schaltung eine Serienschaltung umfasst.

19. Verfahren nach einem der vorausgehenden Ansprüche, wobei die Schaltung eine Parallelschaltung umfasst.

20. Verfahren nach einem der vorausgehenden Ansprüche, wobei die Schaltung an eine äußere Oberfläche eines Gewebekörpers (12) angebracht wird.

21. Heizender/wärmender Gewebeartikel (10), aufweisend:
einen Gewebekörper (12) mit einer ersten und zweiten breiten Oberfläche; und
eine an einer von der ersten und der zweiten breiten Oberfläche angebrachte elektrisch leitfähige Schaltung (16) zum Erzeugen von lokaler Erwärmung des Gewebeartikels bei Anlegen von elektrischem Strom an die Schaltung;
**dadurch gekennzeichnet, dass**
die elektrisch leitfähige Schaltung (16) als eine elektrisch leitfähige Textilie aus leitfähigen Fasern ausgebildet ist, welche in die Form der Schaltung gestanzt wird.

22. Gewebeartikel nach Anspruch 21, wobei der Gewebekörper (12) ein Textilmaterial aufweist, welches ausgewählt ist aus der Gruppe bestehend aus Kulierwirkmaterial, Kettwirkmaterial, gewebtem Material und nicht gewebtem Material; vorzugsweise weist die Gewebeschicht eine glatte Oberfläche, eine raue Oberfläche oder eine gebürstete Oberfläche auf.

23. Gewebeartikel nach Anspruch 21 oder 22, wobei der Gewebeartikel (10) einen Kleidungsartikel umfasst.

24. Gewebeartikel nach einem der Ansprüche 21, 22 oder 23, wobei der Gewebeartikel (10) eine Decke, einen Überwurf, einen Schlafsack oder einen Matratzenschoner umfasst.

25. Gewebeartikel nach einem der Ansprüche 21 bis 24, ferner Haftmittel aufweisend, welches zwischen der Schaltung und dem Gewebekörper eingebracht wird.

26. Gewebeartikel nach einem der Ansprüche 23 bis 25, wobei der Kleidungsartikel (60) einen Artikel aufweist, welcher ausgewählt ist aus der Gruppe bestehend aus Handschuhen, Socken, Pullovern, Jacken, Hemden, Hosen, Hüten, Schuhwerk, Ohrenschützern, Nackenwärmern, medizinischen Klammern, medizinischen Bändern, Kniepolstern, Rückenpolstern und Gelenkpolstern.

27. Gewebeartikel nach einem der Ansprüche 23 bis 26, wobei die Schaltung Flächen (82) mit relativ hohem Widerstand und Flächen (80) mit relativ geringem Widerstand aufweist, um vorgegebene Bereiche mit relativ hoher lokaler Erwärmung und vorgegebene Bereiche mit relativ geringer lokaler Erwärmung bereitzustellen.

28. Gewebeartikel nach Anspruch 27, wobei die Flächen mit relativ hohem und relativ geringem Widerstand Bereiche mit relativ geringer bzw. relativ großer Querschnittfläche aufweisen.

29. Gewebeartikel nach einem der Ansprüche 23 bis 28, wobei der Gewebeartikel (10) einen Kleidungsartikel (60) umfasst, und die Schaltung dazu ausgebildet ist, die Flächen mit relativ hohem Widerstand an Gliedmaßen eines Trägers anliegend anzuordnen, wenn der Kleidungsartikel getragen wird.

30. Gewebeartikel nach einem der Ansprüche 23 bis 29, wobei der Gewebeartikel (10) einen Kleidungsartikel (60) umfasst, und die Schaltung dazu ausgebildet ist, die Flächen mit relativ hohem Widerstand an den Bereichen des Körpers des Trägers anliegend anzuordnen, wo sich Arterien nahe der Hautoberfläche befinden, wenn der Kleidungsartikel getragen wird.

31. Gewebeartikel nach einem der Ansprüche 23 bis 30, weiter aufweisend eine Sperrschicht
(14) zwischen dem Gewebekörper (12) und der Schaltung (16).

32. Gewebeartikel nach Anspruch 31, wobei die Sperrschicht (14), der Gewebekörper (12) und die Schaltung (16) durch Haftmittel (18) miteinander verbunden sind.

33. Gewebeartikel nach einem der Ansprüche 21 bis 32, wobei die Schaltung eine Serienschaltung aufweist.

34. Gewebeartikel nach einem der Ansprüche 21 bis 33, wobei die Schaltung eine Parallelschaltung aufweist.

35. Gewebeartikel nach einem der Ansprüche 21 bis 34, wobei die Schaltung asymmetrisch ist.

36. Gewebeartikel nach einem der Ansprüche 21 bis 35, ferner einen Temperatursensor zum Messen der Temperatur eines Teiles der Schaltung aufweisend.

37. Gewebeartikel nach Anspruch 36, wobei der Temperatursensor eingerichtet ist, um die Temperatur eines ersten Teiles der Schaltung zu messen, und der erste Teil der Schaltung so ausgebildet ist, denselben Widerstand wie ein zweiter Teil der Schaltung aufzuweisen, um zu ermöglichen, dass die Temperatur des zweiten Teils durch Messung der Temperatur des ersten Teils abgeschätzt wird.

38. Gewebeartikel nach Anspruch 36 oder 37, ferner eine Steuerung aufweisend, die dazu eingerichtet ist, den der Schaltung zugeführten Strom in Reaktion auf Änderungen der gemessenen Temperatur anzupassen.

39. Heizender/wärmender Gewebeartikel gemäß Anspruch 21, wobei die leitfähige Textilie eine Form aufweist, die einem ausgewählten Oberflächenbereich eines Körpers eines Trägers entspricht und mit einem oder mehreren Schaltungsbereichen mit relativ hohem Widerstand unter einem oder mehreren Schaltungsbereichen mit relativ niedrigem Widerstand, wobei der eine oder die mehreren Schaltungsbereiche mit relativ hohem Widerstand zur Zuordnung zu einem oder mehreren ausgewählten Heizbereichen des Körpers des Trägers angeordnet sind, die leitfähige Textilie bei Anlegen von elektrischem Strom an die Schaltung eine lokale Erwärmung in dem einen oder den mehreren Schaltungsbereichen mit relativ hohem Widerstand der Schaltung erzeugt, welche auf dem Gewebekörper zum bevorzugten Heizen des einen oder der mehreren Heizbereiche des Körpers des Trägers angebracht ist.

40. Heizender/wärmender Gewebeartikel gemäß Anspruch 21, wobei die leitfähigen Fasern die elektrisch leitfähige Schaltung bilden.

41. Heizender/wärmender Gewebeartikel gemäß Anspruch 21, wobei die leitfähigen Fasern ein Material aufweisen, welches ausgewählt ist aus der Gruppe bestehend aus Kohlenstoff und Polyanilin.

## Revendications

1. Procédé de formation d'un article électrique de tissu chauffant/réchauffant (10), le procédé comprenant :
la fixation d'un circuit électriquement conducteur (16) sur la première ou la seconde surface large d'un corps de tissu (12) pour produire un chauffage localisé du corps de tissu (12) lors de l'application d'un courant électrique audit circuit ; **caractérisé en ce que** :
le circuit électriquement conducteur (16) est formé en découpant à l'emporte-pièce un textile électriquement conducteur de fibres conductrices.

2. Procédé selon la revendication 1 dans lequel ladite étape de fixation comprend la jonction du circuit (16) au corps de tissu (12) par un adhésif (18).

3. Procédé selon l'une quelconque des revendications précédentes comprenant en outre la formation d'un article de vêtement (60) comprenant ledit corps de tissu (12).

4. Procédé selon la revendication 3 dans lequel l'étape de formation comprend le façonnage du circuit pour l'adapter à la forme de l'article de vêtement (60).

5. Procédé selon la revendication 3 ou 4 dans lequel l'article de vêtement (60) comprend un article sélectionné dans le groupe composé des gants, des chaussettes, des pulls, des vestes, des chemises, des pantalons, des chapeaux, des chaussures, des protège-oreilles, des foulards, des corsets médicaux, des bandes médicales, des genouillères, des protections pour le dos et des protections pour articulation.

6. Procédé selon l'une quelconque des revendications précédentes comprenant en outre la configuration du circuit (16) pour comprendre des zones (82) de résistivité relativement plus élevée et des zones (80) de résistivité relativement plus faible pour fournir des régions prédéterminées de chauffage localisé relativement plus élevé et relativement plus faible.

7. Procédé selon la revendication 6, dans lequel les zones prédéterminées (80, 82) de résistivité relativement plus élevée et relativement plus faible sont fournies par la variation de la superficie de la section d'une ou plusieurs régions sélectionnées du circuit (56).

8. Procédé selon la revendication 6 ou 7, dans lequel les zones prédéterminées (80, 82) de résistivité relativement plus élevée et relativement plus faible sont fournies par la variation de la conductivité d'une ou plusieurs régions sélectionnées du circuit (16).

9. Procédé selon la revendication 6, 7 ou 8, dans lequel l'article électrique chauffant/réchauffant (10) est incorporé dans un article de vêtement (60) et le procédé comprend en outre la configuration du circuit pour placer lesdites zones de résistivité relativement plus élevée près des extrémités d'un porteur lorsque l'article de vêtement est porté.

10. Procédé selon l'une quelconque des revendications 9 à 12, dans lequel l'article électrique chauffant/réchauffant est incorporé dans un article de vêtement (60) et le procédé comprend en outre le façonnage du circuit pour placer lesdites zones de résistivité relativement plus élevée près des régions du corps d'un porteur où le flux sanguin est proche de la surface de la peau lorsque l'article de vêtement est porté.

11. Procédé selon l'une quelconque des revendications précédentes dans lequel le corps de tissu (12) comprend un matériau textile sélectionné dans le groupe composé des matériaux à trame tricotée, des matériaux à maille tricotée, des matériaux tissés et des matériaux non tissés ; de préférence, le corps du tissu (12) a une surface lisse, une surface surélevée ou une surface brossée.

12. Procédé selon l'une quelconque des revendications précédentes comprenant en outre l'interposition d'une couche barrière (14) entre le corps de tissu (12) et le circuit (16).

13. Procédé selon la revendication 12 comprenant en outre la fixation d'une surface externe de la couche barrière (14) sur le corps de tissu (12) et la fixation d'une surface interne de la couche barrière (14) sur le circuit (16).

14. Procédé selon la revendication 13 dans lequel lesdites étapes de fixation comprennent la jonction des couches par de l'adhésif (18).

15. Procédé selon l'une quelconque des revendications précédentes comprenant en outre la connexion du circuit à une source électrique (36) pour générer de la chaleur/du chauffage.

16. Procédé selon l'une quelconque des revendications précédentes comprenant en outre l'incorporation de l'article électrique de tissu chauffant/réchauffant (10) à un article de tissu d'ameublement.

17. Procédé selon la revendication 19 dans lequel l'article de tissu d'ameublement comprend une couverture, un couvre-lit, un sac de couchage ou une housse de matelas.

18. Procédé selon l'une quelconque des revendications précédentes dans lequel ledit circuit comprend un circuit en série.

19. Procédé selon l'une quelconque des revendications précédentes dans lequel ledit circuit comprend un circuit parallèle.

20. Procédé selon l'une quelconque des revendications précédentes dans lequel ledit circuit est fixé à une surface externe d'un corps de tissu (12).

21. Article de tissu chauffant/réchauffant (10), comprenant :
un corps de tissu (12) ayant des première et seconde surfaces larges ; et
un circuit électriquement conducteur (16) fixé à la première ou à la seconde surface large pour produire un chauffage localisé de l'article en tissu lors de l'application d'un courant électrique audit circuit ; **caractérisé en ce que** :
le circuit électriquement conducteur (16) est formé comme un tissu électriquement conducteur de fibres conductrices découpées à l'emporte-pièce d'après la forme du circuit.

22. Article de tissu selon la revendication 21 dans lequel le corps de tissu (12) comprend un matériau textile sélectionné dans le groupe composé des matériaux à trame tricotée, des matériaux à chaîne tricotée, des matériaux tissés et des matériaux non tissés ; la couche de tissu a de préférence une surface lisse, une surface surélevée ou une surface brossée.

23. Article de tissu selon la revendication 21 ou 22 dans lequel l'article de tissu (10) comprend un article de vêtement.

24. Article de tissu selon l'une quelconque des revendications 21, 22 ou 23 dans lequel l'article de tissu (10) comprend une couverture, un couvre-lit, un sac de couchage ou une housse de matelas.

25. Article de tissu selon l'une quelconque des revendications 21 à 24 comprenant en outre un adhésif (18) intercalé entre le circuit et le corps de tissu.

26. Article de tissu selon l'une quelconque des revendications 23 à 25 dans lequel l'article de vêtement (60) comprend un article sélectionné dans le groupe composé des gants, des chaussettes, des pulls, des vestes, des chemises, des pantalons, des chapeaux, des chaussures, des protège-oreilles, des foulards, des corsets médicaux, des bandes médicales, des genouillères, des protections pour le dos et des protections pour articulation.

27. Article de tissu selon l'une quelconque des revendications 23 à 26 dans lequel le circuit comprend des zones (82) de résistivité relativement plus élevée et des zones (80) de résistivité relativement plus faible pour fournir des régions prédéterminées de chauffage localisé relativement plus élevé et des régions prédéterminées de chauffage localisé relativement plus faible.

28. Article de tissu selon la revendication 27 dans lequel les zones (80, 82) de résistivité relativement plus élevée et relativement plus faible comprennent des régions de section relativement plus réduite et relativement plus grande, respectivement.

29. Article de tissu selon l'une quelconque des revendications 23 à 28 dans lequel l'article de tissu (10) comprend un article de vêtement (60) et le circuit est configuré pour placer lesdites zones de résistivité relativement plus élevée près des extrémités d'un porteur lorsque l'article de vêtement est porté.

30. Article de tissu selon l'une quelconque des revendications 23 à 29 dans lequel l'article de tissu (10) comprend un article de vêtement (60) et le circuit est configuré pour placer lesdites zones de résistivité relativement plus élevée près des régions du corps du porteur où les artères sont proches de la surface de la peau lorsque l'article de vêtement est porté.

31. Article de tissu selon l'une quelconque des revendications 23 à 30 comprenant en outre une couche barrière (14) entre le corps de tissu (12) et le circuit (16).

32. Article de tissu selon la revendication 31 dans lequel la couche barrière (14), le corps de tissu (12) et le circuit (16) sont joints par un adhésif (18).

33. Article de tissu selon l'une quelconque des revendications 21 à 32 dans lequel ledit circuit comprend un circuit en série.

34. Article de tissu selon l'une quelconque des revendications 21 à 33 dans lequel ledit circuit comprend un circuit parallèle.

35. Article de tissu selon l'une quelconque des revendications 21 à 34 dans lequel ledit circuit est asymétrique.

36. Article de tissu selon l'une quelconque des revendications 21 à 35 comprenant en outre un capteur de température pour mesurer la température d'une partie du circuit.

37. Article de tissu selon la revendication 36 dans lequel ledit capteur de température est configuré pour mesurer la température d'une première partie du circuit et la première partie du circuit est configurée pour avoir la même résistance qu'une seconde partie du circuit, pour permettre à la température de la seconde partie d'être estimée en mesurant la température de la première partie.

38. Article de tissu selon la revendication 36 ou 37 comprenant en outre une unité de commande configurée pour régler l'énergie fournie au circuit en réponse aux modifications de la température mesurée.

39. Article de tissu chauffant/réchauffant selon la revendication 21 dans lequel le textile conducteur a une forme correspondant à une région de surface sélectionnée du corps d'un porteur, avec une ou plusieurs régions du circuit de résistivité relativement plus élevée parmi une ou plusieurs régions du circuit de résistivité relativement plus faible, l'une ou les régions du circuit de résistivité relativement plus élevée étant positionnées pour corrélation avec une ou plusieurs régions de chauffage sélectionnées du corps du porteur, le textile conducteur, lors de l'application d'un courant électrique au circuit, produisant un chauffage localisé dans l'une ou les régions du circuit de résistivité relativement plus élevée du circuit fixé sur le corps de tissu pour chauffage préférentiel de la ou des régions de chauffage sélectionnées du corps du porteur.

40. Article de tissu chauffant/réchauffant selon la revendication 21 dans lequel les fibres conductrices forment le circuit électriquement conducteur.

41. Article de tissu chauffant/réchauffant selon la revendication 21 dans lequel les fibres conductrices comprennent un matériau sélectionné parmi le groupe composé de carbone et de polyaniline.
